# EUROPEAN PATENT SPECIFICATION

(11) **EP 2 709 686 B1**
(45) Date of publication and mention of the grant of the patent: **15.03.2017**
(21) Application number: 12785264.8
(22) Date of filing: 11.05.2012
(51) Int. Cl.: A61M 1/00, A61B 17/22, A61M 3/00, A61B 17/50, A61M 27/00

(54) **REMOTELY CONTROLLED SUCTION/IRRIGATION FOR SURGERY**
FERNGESTEUERTE ANSAUGUNG/BEWÄSSERUNG FÜR DIE CHIRURGIE
ASPIRATION / IRRIGATION TÉLÉCOMMANDÉES POUR CHIRURGIE

(30) Priority: 13.05.2011 US 201161485833 P
(43) Date of publication of application: 26.03.2014
(73) Proprietor: Vascular Technology, Inc., Nashua, New Hampshire 03062 (US)
(72) Inventor: DOUGLAS, Gary, Billerica, Massachusetts 01821 (US); MARTONE, Stephen, Nashua, New Hampshire 03062 (US); REGAN, David, Pelham, New Hampshire 03076 (US); SRIVASTAVA, Nilendu, Hollis, New Hampshire 03049 (US); SUCHDEV, Rachana, Hollis, New Hampshire 03049 (US)
(74) Representative: Petraz, Gilberto Luigi
(86) International application number: PCT/US2012/037516
(87) International publication number: WO 2012/158516

(56) References cited:
- WO-A2-03/006084
- WO-A2-2010/138521
- WO-A2-2010/138521
- US-A1- 2004 133 165
- US-A1- 2007 005 002
- US-A1- 2007 005 002
- US-A1- 2008 154 183
- US-A1- 2008 262 308
- US-A1- 2010 312 186
- US-A1- 2010 312 186
- US-B1- 7 083 601
- US-B1- 7 083 601

## Description

### CROSS REFERENCE TO RELATED APPLICATIONS

This application claims benefit to US Provisional Application No. 61/485833 filed on May 13, 2011.

### FIELD OF THE INVENTION

The invention relates to suction/irrigators for surgery and more particularly, to a remotely controlled suction irrigation device for use in surgical procedures.

### BACKGROUND OF THE INVENTION

In surgical procedures, it is often necessary to irrigate sterile solutions into and aspirate bodily or irrigant fluids out of the surgical field. Traditionally, suction/irrigation devices have been designed to function as hand held tools intended for use by the operating surgeon or an operative assistant. These hand held devices typically incorporate a valve mechanism which the surgeon manipulates manually to control suction and irrigation functions. A common valve configuration in use today is known as a "trumpet valve". The trumpet valve consists of a button which can be manually depressed by the operator against a compression spring to engage a valve barrel; manual depression of the suction key allows for aspiration through the device, while depression of the irrigation button allows for the irrigation of fluids through the device.

Recently, robot assisted surgery has been increasingly employed by surgeons to perform technically challenging procedures in a minimally invasive fashion. In robot assisted surgery, the operating surgeon sits at a robotic console and remotely controls robotic arms within the surgical field to perform the surgery. An assistant surgeon is present at the patient's side to manipulate tools that cannot be controlled robotically. One such tool is the suction/irrigation probe.

Suction/irrigation devices that are currently employed in robot assisted surgery are devices that were designed for laparoscopic surgery. The assistant surgeon inserts a rigid laparoscopic suction/irrigation probe through an accessory port and then manually manipulates this probe within the surgical field. As mentioned above, the control buttons on this probe consist of a dual trumpet valve which must be manually pressed and depressed by the assistant surgeon, and cannot be manipulated by the lead surgeon seated at the robotic console. Thus, the lead surgeon must continuously instruct the assistant as to when, where and how to utilize the device throughout the surgical case.

The concept of a "hand-held" suction/irrigation device can pose problems outside the realm of robot assisted surgery as well. In many open surgical procedures, the operating surgeon will often require an assistant to perform suction and/or irrigation within the surgical field while the surgeon is performing other surgical maneuvers (ex. suturing or dissection). In these instances, the surgeon will often have an instrument in each hand, preventing him/her from operating the valves on a traditional suction/irrigation device. In these instances, as well as in robot assisted surgery, it would be ideal for the surgeon to be able to place an appropriately designed suction/irrigation device within the surgical field and control the operation of this device in a "hands-free" or remote fashion.

An additional limitation posed by the use of trumpet valves or similarly actuated valve mechanisms is the propensity of these valves to become obstructed with particulate materials. The design of such valves not only limits the luminal size at the valve barrel, but also creates unnecessary surfaces/edges within the lumen that can cause turbulence and promote obstruction; these factors contribute to the creation of a "bottleneck" at the valve mechanism. An ideal control valve would operate externally to the flow path and allow for complete opening of the inner lumen, thus minimizing the potential for obstruction of flow.

Yet another limitation imposed by the valvular mechanisms that are currently used in suction/irrigation devices today is that they impede the ability to manipulate the flow path in instances of obstruction. Frequently, obstruction of a suction/irrigation device will occur at the valve assembly; however, most valve assemblies are either integrated into the flow stream or inaccessible to manipulation because of device design. A valve mechanism that would allow a user to removably manipulate the tubing at the valve/tubing interface to clear a nidus of obstruction or otherwise troubleshoot clotting at the valve interface would be an advance in the field.

Furthermore, the rigid design of the distal portion of a suction/irrigation probe inherently limits the operating range of this device. Laparoscopic devices are inserted into the operative field via a fixed port. The fixed port and the rigidity of the probe only allow for a limited range of motion for the probe within the surgical field. Thus, there are areas within the surgical field in which the rigid suction/irrigation probe cannot be employed. In these areas, alternative ports must be used to properly place the rigid probe and thus allow for suction/irrigation capability - this not only adds to the complexity of the case, but may also necessitate an additional surgical incision for the patient. This limitation is problematic not only in robot assisted surgery, but in other forms of minimally invasive/laparoscopic surgery as well.

In addition, currently employed suction/irrigation devices are typically single use devices and must be disposed of at significant cost. To reduce cost, an ideal disposable suction/irrigation device would limit the components that became contaminated with use, thus necessitating replacement of only a limited number of such components.

Moreover, suction and irrigation capabilities are only required intermittently during a surgical procedure. Thus, while in robot assisted procedures it may be possible to provide a robotic arm that gives the console surgeon the ability to apply suction and/or irrigation, this necessitates dedication of an arm to these functionalities. A suction/irrigation device that could be controlled by the console surgeon without necessitating dedicated use of a robotic arm would be an advance in the field.

US 2007/005002 A1 (2007-01-04) discloses robotic surgical instruments for irrigation, aspiration and blowing. WO 2010/138521 A2 (2010-12-02) discloses an endoscopic system with suction and irrigation.

What is needed, therefore, is a flexible suction/irrigation device which can be remotely controlled by a surgeon, including a surgeon who may be operating from a robotic console, which also ideally optimizes the valvular mechanism to minimize the incidence of obstruction and allow for clearance of the obstruction, and minimizes disposable components to minimize cost without compromising patient safety.

### SUMMARY OF THE INVENTION

One embodiment of the present invention provides a system for the movement of fluids into and out of a surgical field with a control module manipulated by a user; a valve controlled by the user via the control module, the valve controlling flow of a fluid; and a tube set having a proximal branch and a distal branch, wherein the proximal branch is opened and closed by the valve and at least a portion of the distal branch being flexible and a distal tip of the distal branch configured for manipulation within a surgical field. The valve is disposed at a distance to the surgical field and allows free access to tube by the user. Separate proximal branches for suction, irrigation, and insufflation are provided. One or more distal branch may be provided. Distal branches may be equipped with additional valves whereby they may be closed to allow flushing of the system during the surgical procedure to remove blockages in the suction line.

Another embodiment of the present invention provides such a system wherein the tip is a rigid tip.

A further embodiment of the present invention provides such a system wherein the tip has a length not longer than 2 inches.

Still another embodiment of the present invention provides such a system wherein the tip comprises a porous surgical mat.

A still further embodiment of the present invention provides such a system further comprising a remote controller by which the user manipulates the control module.

Yet another embodiment of the present invention provides such a system wherein the remote controller is selected from the group of remote controllers consisting of voice controllers, foot pedals, pneumatically activated controls, wirelessly transmitted controllers and touch sensitive hand controllers.

A yet further embodiment of the present invention provides such a system wherein the remote controller is integrated into a robotic surgical system control console.

Even another embodiment of the present invention provides such a system wherein valve in the valve is a solenoid valve.

An even further embodiment of the present invention provides such a system wherein the solenoid valve comprises a solenoid pinch valve.

Still yet another embodiment of the present invention provides such a system further comprising a pump or regulator whereby the flow of fluid is pressurized.

A still yet further embodiment of the present invention provides such a system wherein the tip of the distal branch is repositionable by a robotic arm.

Even yet another embodiment of the present invention provides such a system wherein the tip of the distal branch is repositionable by a surgical instrument.

An even yet further embodiment of the present invention provides such a system further comprising a second distal branch coupled to the proximal branch and terminating in a laparoscopic suction irrigation probe.

Even still another embodiment of the present invention provides such a system further comprising a valve whereby the distal branch may be occluded.

One embodiment of the present disclosure provides a flexible suction/ irrigation probe, the probe comprising: a distal tip having a plurality of porosities for the passage of fluids and being configured to be manipulated by a surgical robot; and a flexible tube coupled to a proximal end of the distal tip.

Another embodiment of the present disclosure provides such a probe, the probe further comprising a vacuum source coupled to the tube.

A further embodiment of the present disclosure provides such a probe, the probe further comprising an irrigation source coupled to the tube.

Still another embodiment of the present disclosure provides such a probe further comprising a compressed air source coupled to the tube.

A still further embodiment of the present disclosure provides such a probe further comprising a valve whereby the lumen is occluded.

Yet another embodiment of the present disclosure provides such a probe further comprising a check valve disposed within the tube between the distal end and an irrigation source.

One embodiment of the present invention provides a fluid control valve for use in suction, insufflation and irrigation of a surgical field, the valve comprising: a remote control unit operated by a user; a fluid line accessible to the user during a surgical procedure and running from a fluid source to the surgical field, and, opened and closed by a valve controlling the passage of fluid through a lumen of the fluid line; the valve disposed remotely from a surgical field, and remotely controlled by the remote control unit.

Another embodiment of the present invention provides such a fluid control valve, wherein the valve is a pinch valve disposed around the fluid line such that when the pinch valve is activated, the pinch valve compresses the fluid line and occludes the lumen, the pinch valve being configured to avoid turbulence and clotting in fluid passing through the lumen.

A further embodiment of the present invention provides such a fluid control valve wherein the fluid source is a fluid source chosen from the fluid sources consisting of vacuum sources, compressed gas sources, carbon dioxide sources, irrigation liquid reservoirs, and saline supplies.

Still another embodiment of the present invention provides such a fluid control valve further comprising a second pinch valve controlling fluid flow through a second fluid line, the second pinch valve being configured to be open only when the pinch valve is closed.

The features and advantages described herein are not all-inclusive and, in particular, many additional features and advantages will be apparent to one of ordinary skill in the art in view of the drawings, specification, and claims. Moreover, it should be noted that the language used in the specification has been principally selected for readability and instructional purposes. The invention is defined by the claims.

### BRIEF DESCRIPTION OF THE DRAWINGS

Figure 1 is a block diagram illustrating a flexible suction irrigator configured in accordance with one embodiment of the present invention and manipulable by a robotic surgical grasper.
Figure 2 is a block diagram illustrating a flexible suction irrigator system configured in accordance with one embodiment of the present invention.
Figure 3 is a block diagram illustrating a fluid supply hookup for a flexible suction irrigator configured in accordance with one embodiment of the present invention.
Figure 4 is a block diagram illustrating a valvular unit of a flexible suction irrigator system configured in accordance with one embodiment of the present invention
Figure 5 is a block diagram illustrating a valvular unit of a flexible suction irrigator system configured in accordance with one embodiment of the present invention having pressure regulators and an insufflation line.
Figure 6 is a block diagram illustrating a flexible suction irrigator system configured in accordance with one embodiment of the present invention.
Figure 7 is a block diagram illustrating a detail of a flexible suction irrigator system valvular unit configured in accordance with one embodiment of the present invention.
Figures 8A-8G are block diagrams illustrating various tips for use with a flexible suction irrigator configured in accordance with one embodiment of the present invention.
Figure 9 is an exploded perspective view of a suction irrigator tip configured in accordance with one embodiment of the present invention.
Figure 10 is a perspective view of a suction irrigator surgical platform configured in accordance with one embodiment of the present invention.
Figure 11 is a perspective view of a suction irrigator tubing set configured in accordance with one embodiment of the present invention.
Figure 12 is a perspective view of a suction irrigator tubing set configured in accordance with one embodiment of the present invention with an assistant surgeon suction probe.
Figure 13 is a perspective view of a suction irrigator hand control configured in accordance with one embodiment of the present invention.

### DETAILED DESCRIPTION

One embodiment of the present invention provides a suction/irrigation device for use during surgery that can be manipulated and controlled by a surgeon in a hands-free or remote fashion. The device consists of a flexible distal probe which can be brought into the operating field and a valvular unit which allows for the remote operation of suction and irrigation functions by the surgeon.

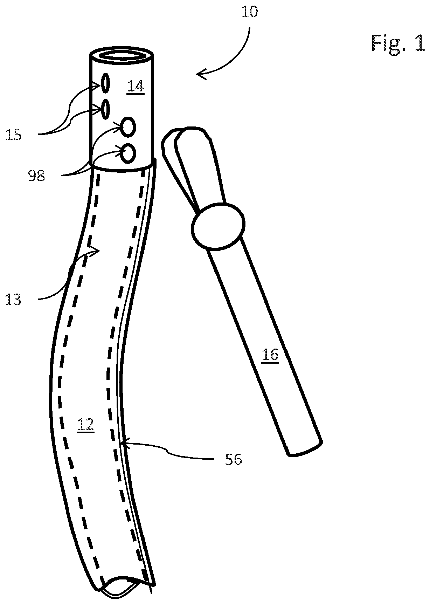

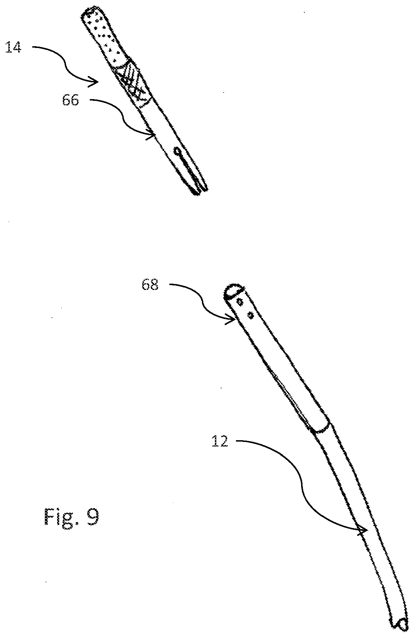

The flexible distal probe **10** illustrated in **Figure 1** consists of flexible tubing **12** with at least one lumen **13** that can be inserted into a surgical field and a graspable tip **14**. The tubing **12** can be configured for insertion through a surgical port for use in minimally invasive or robot-assisted surgery. The graspable tip **14** in one embodiment is as simple as a hollow, rigid cylindrical attachment to the tubing with suction relief holes **15** which allows the surgeon to grasp the tip **14** with a surgical tool **16** and manipulate the flexible probe easily. The surgical tool **16** could comprise a tool integrated within a robotic arm. Tip **14** could be attached to tubing **12** using an adhesive (in one embodiment the adhesive is biocompatible), a coupling sleeve, or any other mechanical means of attachment. In an alternative embodiment, as illustrated in **Figure 9**, the tip **14** may be configured to be detachably affixed to the tubing **12** with a compression joint, the joint being made up of complementary male **66** and female **68** fittings. Other detachable means of connecting tip **14** to tubing **12** could also be employed.

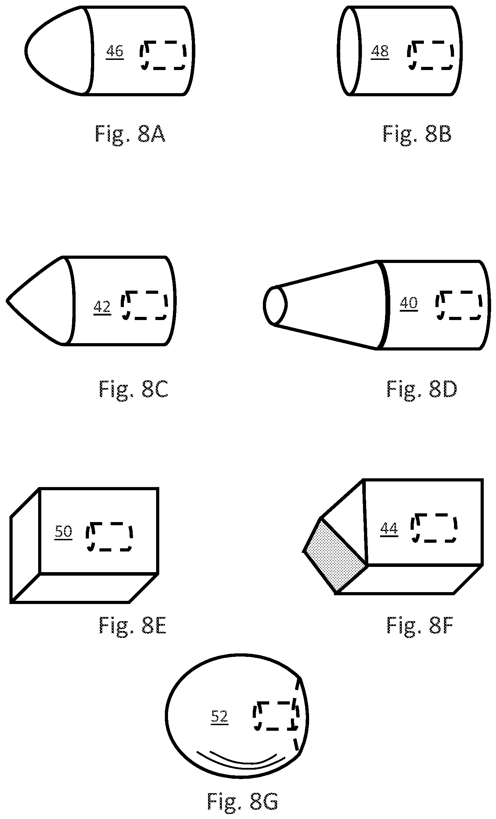

The graspable tip **14** can also take the form of a porous sintered or foam tip **14** which would provide for greater dissection capability of the invention. Possible tips may include a tip such as that described in US App. No. 12/797,067 filed June 9, 2010 which is incorporated herein by reference for all purposes. Such tips, as illustrated in **Figures 8A-8G** can be conic **42**, frustro-conic **40**, wedge shaped **44**, cylindrical **48**, spherical **52**, cubic **50** or some combination thereof **46**. Alternatively, the graspable tip **14** could be fashioned by modifying the end of the flexible tubing **12** by processes such as shaping the tubing to allow for greater purchase on the tubing by the grasper **16** or by the introduction of suction relief holes through the wall of the tube, allowing for ease of use with a robotic arm without a separate tip.

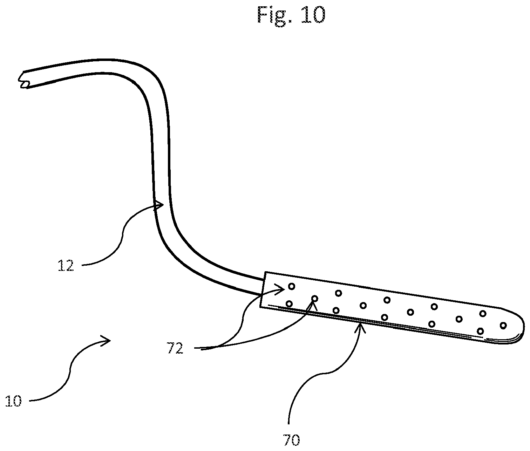

In another embodiment of the present invention, the tip **14** could comprise a porous surgical mat as illustrated in **Figure 10**. In this embodiment, the flexible distal probe **10** consists of flexible tubing **12** and a porous, low profile, rigid, semi-rigid or flexible manifold **70** which can be constructed of any biocompatible material including but not limited to PVC, polytetrafluoroethylene (PTFE), polyurethane, polycarbonate, polyether ether ketone (PEEK), polyamides, nylon, silicone and polyether block amide (PEBAX). Processes which could be used to fabricate such a surgical mat include but are not limited to extruding, molding, forming, machining, selective laser sintering, stereolithography, and fused deposition modeling. This manifold/mat **70** is designed to be placed under or behind an anatomical structure(s) within the surgical field. The porosities **72** within the mat **70** allow for suction and irrigation to be delivered via the tubing **12**, through the mat **70** and into the surgical field. The semi-rigid nature of the manifold **70** provides a stable surface upon which to perform surgical maneuvers including but not limited to anastomoses of microvasculature, nerve fascicles, and ligaments in reconstructive procedures, rejoining of ligated vas deferens tubules in vasectomy reversal procedures, and ligation of aberrant vasculature in varicocelectomy procedures.

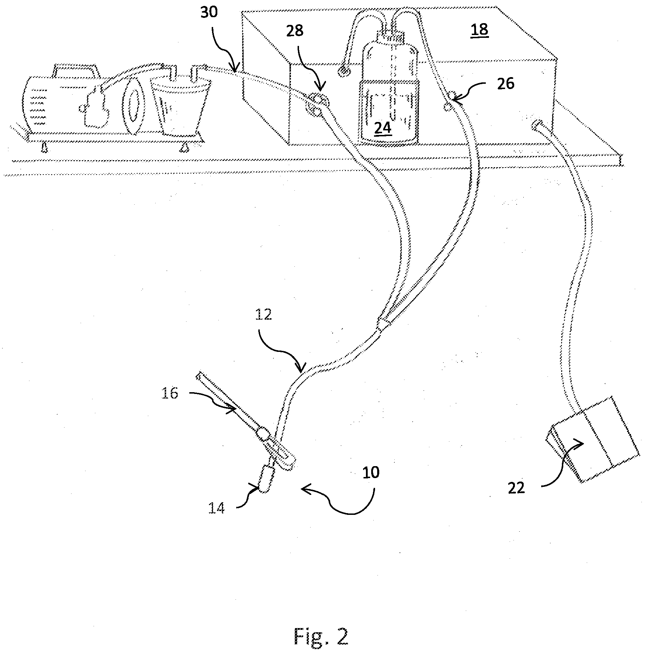

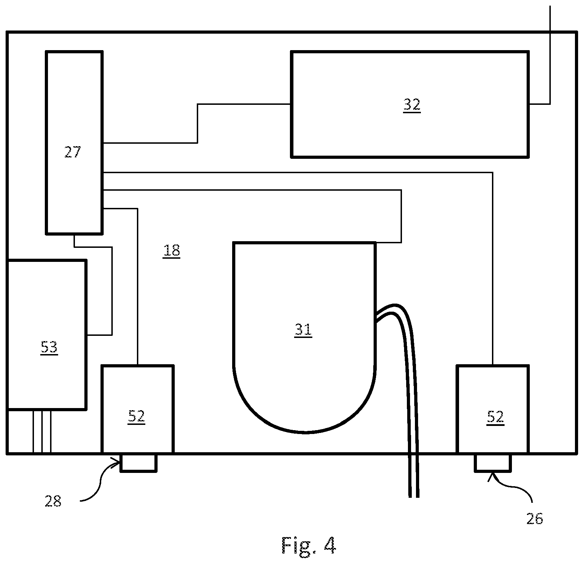

A valvular unit **18** as illustrated in **Figure 2** may be controlled through wired, wireless, or mechanical means. In one embodiment, the user may control the valvular unit **18** with a foot pedal **22**. The user input via the valvular unit **18** allows the valvular unit **18** to actuate a supply of sterile saline or other irrigation fluid **24** and two valves **26, 28**. A first valve **26** controls the flow of irrigation fluid from the supply source **24**, while that of the second valve **28** controls **30** a line running from a vacuum source such as a vacuum pump or vacuum line. System **18** as seen in **Figure 4** may also be equipped with power supply **32** supplying power to the irrigation fluid supply pump **31** and actuators for the valves **26, 28**. A bus bar or terminal block **27** receives power from the power supply 32 and distributes power to various components the valvular unit **18**.

The valvular unit **18** sits outside of the surgical field. The valvular unit **18** of one embodiment uses valves **26, 28** that are solenoid pinch valves **52** which are used to turn the suction and irrigation functions on and off. One skilled in the art will appreciate that other valves or switches may be employed, such as, mechanical solenoid valves. Power switches **53** may be provided to allow the user to turn on the valvular unit **18** and to switch between running and setup modes. The pinch valves can be controlled via various mechanisms including (but not limited) to a foot pedal **22**, additional buttons at the robotic console finger controls, buttons **98** at and/or on the distal probe tip **12**, as in **Fig 1**, (which may require wiring **56** back to the control unit along the flexible tubing), voice activation or any other actuation signal communication forms.

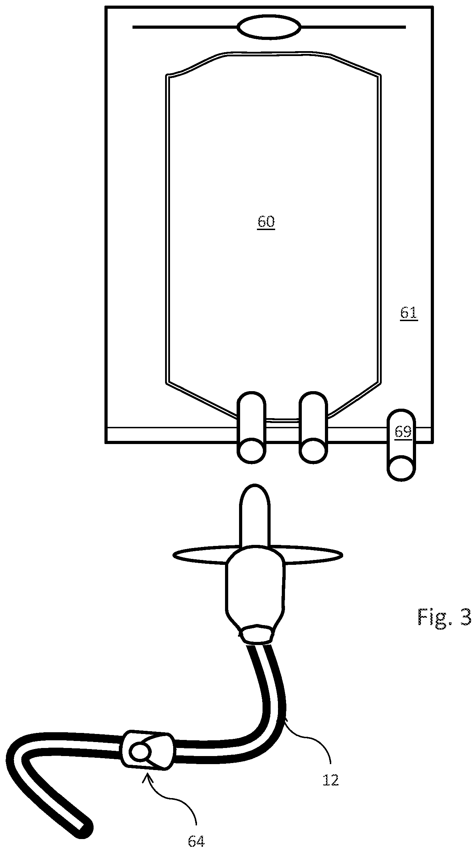

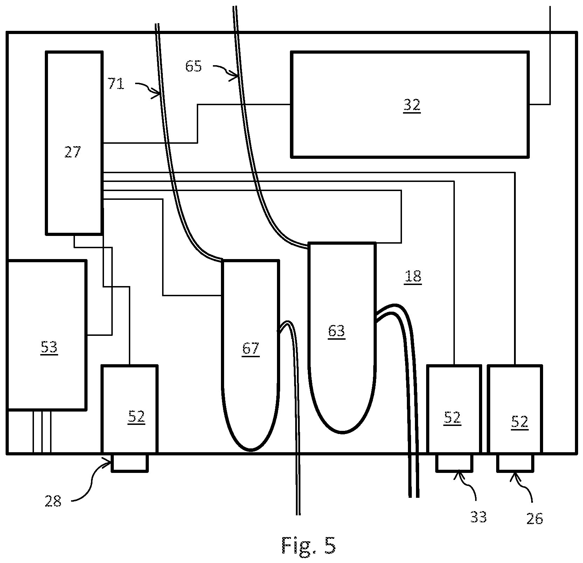

In addition, the valvular unit irrigation fluid supply **24** may include a pumping mechanism (ex. air pump) **31** to allow for "power" irrigation and/or gravity irrigation in the event of power failure. Alternatively, such a pump could be housed in a unit separate from the unit that allows for actuation of suction and/or irrigation capabilities. In one embodiment of the present invention, a detail of which is illustrated in **Figure 3**, the system may be configured to provide irrigant from a fluid bag **60** (ie. such as that used for intravenous infusions) or other container separate from the valvular unit **18**. Elevation of the fluid bag would allow for gravity fed irrigation. Alternatively, an inflatable pressure cuff **61** could be placed around the fluid bag and inflated via the air pump housed in the valvular unit **18** to allow for pressurized/powered irrigation. In another embodiment of the present invention, as shown in **Figure 5** the valvular unit **18** would comprise a connection **65** to an external pressurized air source such as the hospital central air supply. The valvular unit **18** would house a pressure regulator **63** with or without a safety pressure relief valvewhich would allow for the delivery of a constant air pressure to the inflatable pressure cuff via connector **69**. A similar system may be employed to provide insufflation capability wherein a gas, such as compressed air or carbon dioxide is the fluid supplied , from a gas supply **71** through a regulator **67** to a proximal branch and ultimately to the surgical field. In such a valvular unit, an additional valve 33 may be provided to control gas from the gas regulator **67**. As used throughout the specification, fluid is used to indicate a substance in either a gas or liquid phase and any particulates or sold masses contained therein.

One embodiment of the present invention further provides at least one check valve **64** disposed within said flexible tube to prevent fluids from returning to the pump or from evacuated fluids being returned to the surgical field

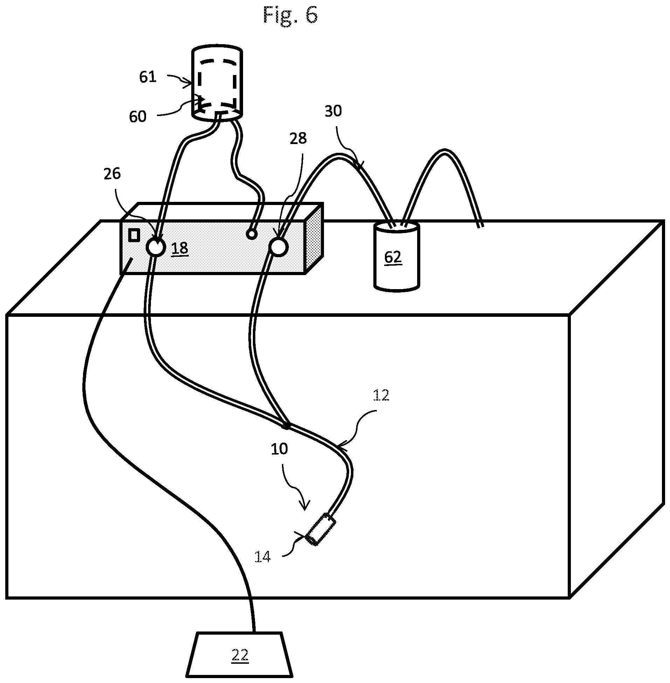

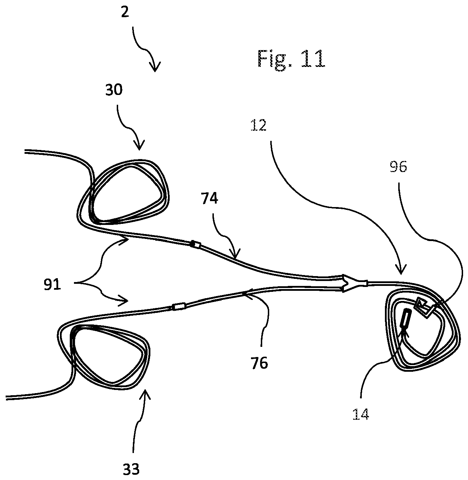

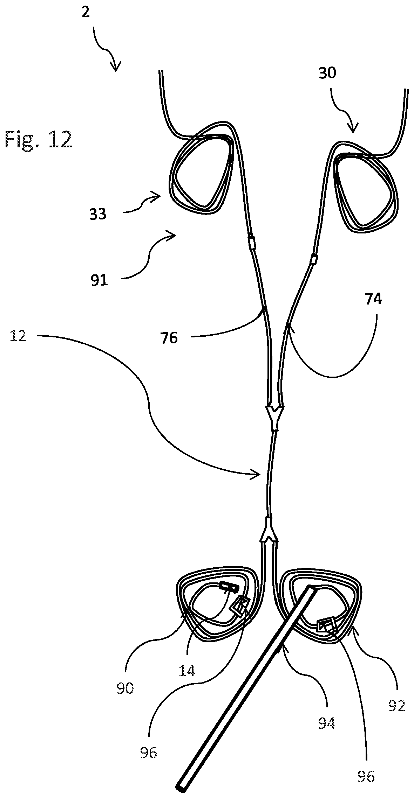

As illustrated in **Figure 6**, the tubing set **2**, illustrated in more detail in **Figures 11** **and** **12**, of the invention has a distal branch of tubing **12** (and possibly wiring, not shown) and a proximal branch of tubing that comes back to the valvular unit **18**, passes through the solenoid pinch valves **26, 28** and then connects to both a suction source/container **62** and an irrigation source **60**. In this manner, only the tubing set **2**, and the container **62** for collection of the aspirant is exposed to contaminants. All other components of the valvular unit **18**, including the solenoid pinch valves **26, 28**, the irrigation pump **31** and/or pressure regulators **63, 67** and the power supply **32** can be reused. Such a design not only reduces costs but also minimizes the need to dispose of bulky disposable pump apparatus. Furthermore, the use of pinch valves is less prone to blockage than trumpet valves used in known systems, as pinch valves do not come into contact with the lumen and fluid flow path, thus minimizing turbulence within the flow stream.

As shown in **Figures 11** **and** **12**, the proximal branch **91** of tubing set 2 may comprise sections of compliant tubing (**74, 76**), such as silicone tubing, allowing for compression by the pinch valves. A tubing set **2** of one embodiment has at least one a distal branch configured for manipulation within the surgical field and a proximal branch outside of the surgical field and configured for connection with the valvular mechanism of the valvular unit, tubing sections **74** and **76** can be accessed and manipulated during a surgical procedure. For example, if tubing section **74** was to become clotted with blood clots aspirated during surgery, a user or assistant could place the unit in "setup" mode via control switch **53** (which would open both solenoid valves **26** and **28**), lift pliant tubing section **74** out of the valvular mechanism, and mechanically dissolve the clot by applying manual pressure to the outer tubing surface to restore the suctioning functionality. Alternatively, a valve **93** or pinch clamp **96could** be closed and both solenoid valves **26** and **28** could be opened thereby forcefully flushing the clot from the selected area of the tubing set **2**.

Tubing **12**, according to one embodiment of the present invention may be tubing made from PVC, silicone or C-flex type tubing or other tubing with desired biocompatibility, flexibility and resistance to leakage or rupture even after a high number of flexure cycles. As illustrated in **Figure 11**, conformable sections of tubing **74, 76** may be inserted within tubing of less compressible material to allow better action of valvular mechanisms incorporated into the system.

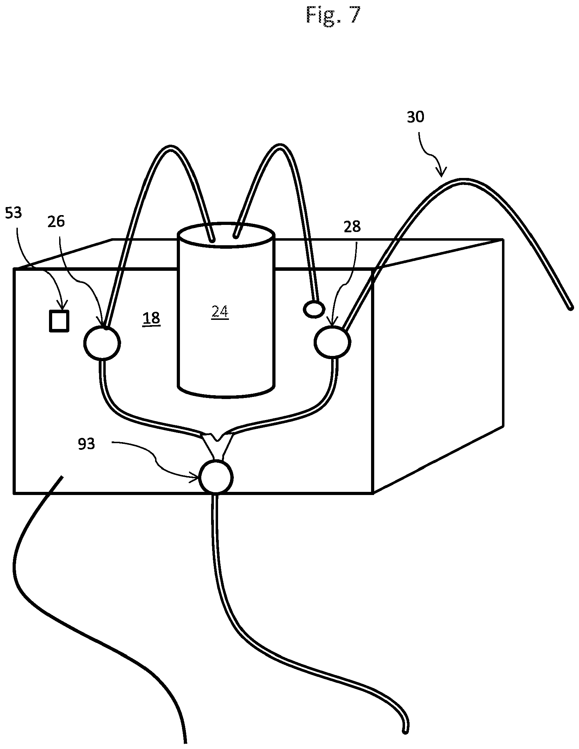

In one embodiment of the present invention, the placement of the valvular mechanism away from the surgical field also allows a user other than the surgeon to control suction, irrigation or blowing functions when necessary. In such an embodiment, valves **26** and **28** would comprise solenoid valves with push buttons which could be manually depressed to allow for opening and closing of the valves. This again would be especially important if a clot were to obstruct the flow of fluid within the system. Taking again the example above, if a clot was to develop in tubing section 74 thus prohibiting suctioning, nursing staff in the operating room could troubleshoot this problem via manual manipulation of the valvular unit. Besides employing the maneuver mentioned above, the nursing staff could also close off the pinch valve **96** or as in **Figure 7**, an additional valve **93** on said valvular unit **18** that controls the distal branch, and then simultaneously press both the suction and irrigation control buttons on the valvular unit. This would force pressurized irrigant to flow preferentially from tubing limb **33** through tubing section **76** and up through tubing limb **30** via tubing section **74**. This "purging" of the system could allow nursing staff to clear clots within the tubing set and particularly at the valvular interfaces if such clots were to develop. In an alternative embodiment, valve 93 could also be remotely controlled thus allowing a surgeon operating from a robotic console to purge the system remotely.

In one embodiment of the present invention, the console surgeon uses the robotic arms to grasp and manipulate the distal probe tip **14**, and uses the foot pedal **22** (or other actuation controls such as voice activation, controls integrated into the robotic console, or buttons at the distal probe tip, pneumatically activated controls, and wirelessly transmitted controllers) to apply suction or irrigation via control of the solenoid valves. This design allows the console surgeon to independently control suction, irrigation and insufflation/blowing functions without the need for an assistant. In addition, the flexible design of the suction/irrigation probe allows the console surgeon to apply suction and/or irrigation to areas within the surgical field that would have been inaccessible with a rigid probe inserted via a fixed port. This flexible design also allows for complete freedom of motion at the probe tip, enabling the user to articulate the tip 180 degrees such that suction or irrigation could be aimed in a direction opposite to the orientation of the probe. Tubing materials such as braided or reinforced tubing could be used to allow for maximal "bending" of the probe tip without causing the formation of occlusive kinks within the tube lumen. This freedom of motion is especially useful in minimally invasive procedures when the suction/irrigation probe and robotic/laparoscopic camera are oriented/inserted from similar positions or angles within the surgical field. In these instances, complete freedom of motion at the probe tip would allow the surgeon to clean a soiled camera lens by aiming irrigation in a "backwards" direction.

In another embodiment of the present invention, a user in the room may, directly control the valvular unit, while allowing the console surgeon to manipulate the position of the tip within the surgical field. In an alternative embodiment of the present invention as seen in **Figure 12**, the tubing set **2** is configured to provide two distal limbs **90, 92** such that suction and irrigation functionalities can be utilized by two surgeons using the same valvular unit. In one embodiment, limb **92** is equipped with a laparoscopic suction irrigator tip **94** for manual manipulation by an assistant surgeon, while limb **96** is equipped with a distal tip **14** for remote manipulation by a robotic surgeon using robotic graspers. Other tip types could be chosen for each of the distal limbs. Additional limbs **90** and **92** could be connected via a Y connector or other appropriate connector to form distal branch **12** of tubing set **2**. In such an embodiment, pinch clamps **96** may be provided to prevent suction or irrigation to occur through an undesired tip. Pinch clamps **96** may be manual clamps as shown in **Figure 12**, or may be powered as with valve **93** in **Figure 7**.

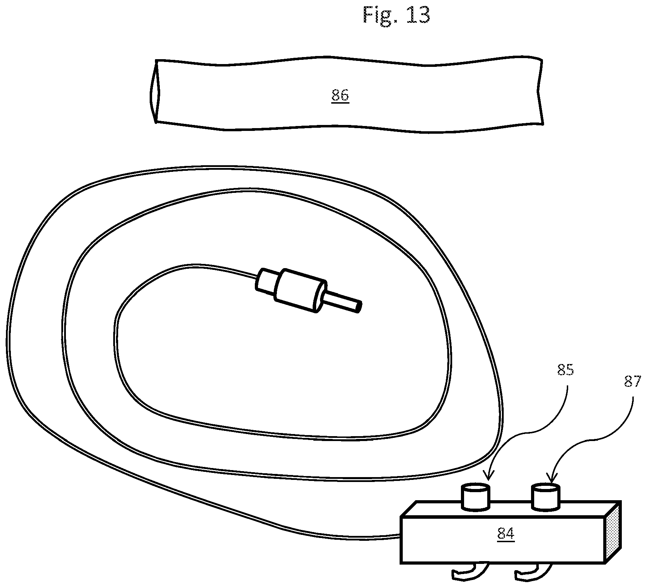

The laparoscopic suction irrigator tip **94**, in one embodiment, could comprise a rigid tube with distal suction relief holes configured for insertion into a laparscopic port. In such an embodiment, a hand control **84** such as that illustrated in **Figure 13** may be used to allow the assistant surgeon to operate the valvular unit. Hand control unit **84** could comprise a small clip-on manifold with push buttons **85** and **87** which could connect to valvular unit **18** via wire **89**. Depression of buttons **85** and **87** would allow the assistant surgeon to open and close valves **26** and **28** on valvular unit **18**. The hand control **84** may be provided sterile or may be covered in a sterile disposable plastic sleeve **86** and may be configured for reuse or single use. Other means by which the assistant surgeon could operate the valvular unit **18** include but are not limited to a second foot pedal connection at the control unit, voice activation or any other actuation signal communication forms.

The foregoing description of the embodiments of the invention has been presented for the purposes of illustration and description. Each and every page of this submission, and all contents thereon, however characterized, identified, or numbered, is considered a substantive part of this application for all purposes, irrespective of form or placement within the application. This specification is not intended to be exhaustive or to limit the invention to the precise form disclosed.

## Claims

1. A system for the movement of fluids into and out of a surgical field, the system being **characterized in that** it comprises:
a control module manipulated by a user;
a valvular unit (18) positioned outside said surgical field and comprising two valves (26, 28) controlled by said user via said control module, said valve unit (18) controlling flow of a fluid; and
a tube set (2) having a proximal branch and a distal branch, wherein said proximal branch is opened and closed by said valve unit (18), passes through said valves (26, 28) and is connected to at least a fluid source (60), and at least a portion (12) of said distal branch being flexible and configured for insertion through a surgical port, and a distal tip (14) of said distal branch configured to be grasped and manipulated with a surgical tool (16) within a surgical field.

2. The system of claim 1 wherein said tip (14) is a rigid tip.

3. The system of claim 2 wherein said tip (14) has a length not longer than 5,08 cm (2 inches).

4. The system of claim 1 wherein said tip (14) comprises a porous surgical mat (70).

5. The system of claim 1 further comprising a remote controller by which said user manipulates said control module.

6. The system of claim 5 wherein said remote controller is selected from the group of remote controllers consisting of voice controllers, foot pedals, pneumatically activated controls, wirelessly transmitted controllers and touch sensitive hand controllers.

7. The system of claim 1 wherein valve in said valve (18) is a solenoid valve.

8. The system of claim 7 wherein said solenoid valve comprises a solenoid pinch valve (52).

9. The system of claim 1 further comprising a pressurized gas supply (71) whereby said flow of fluid is pressurized.

10. The system of claim 1 wherein said tip (14) of said distal branch is repositionable by a robotic arm.

11. The system of claim 1 wherein said tip (14) of said distal branch is repositionable by a surgical instrument (16).

12. The system of claim 1 further comprising a second distal branch coupled to said proximal branch.

13. The system of claim 1 further comprising a valve whereby said distal branch may be occluded.

14. A fluid control valve for use in suction, insufflation and irrigation of a surgical field, said valve being **characterized in that** it comprises:
a remote control unit operated by a user;
a flexible fluid line (12) configured for insertion through a surgical port, running between a fluid source to said surgical field, and, opened and closed by said valve controlling the passage of fluid through a lumen (13) of said fluid line;
an interface between said valve and said fluid line being accessible to said user during operation; and
said valve disposed external to a sterile surgical field, and remotely controlled by said remote control unit.

15. The fluid control valve of claim 14, wherein said valve is a pinch valve (26).

16. The fluid control valve of claim 14, wherein said fluid source is a fluid source chosen from the fluid sources consisting of vacuum sources, compressed gas sources, carbon dioxide sources, irrigation liquid reservoirs, and saline supplies.

17. The fluid control valve of claim 14, further comprising a second pinch valve (28) controlling fluid flow through a second fluid line.

## Patentansprüche

1. System zum Bewegen von Fluiden in ein und aus einem Operationsfeld, wobei das System dadurch charakterisiert ist, dass es Folgendes umfasst:
ein durch einen Benutzer manipuliertes Steuermodul,
eine Ventileinheit (18), welche außerhalb des Operationsfeldes angeordnet ist und zwei durch den Benutzer via des Steuermoduls gesteuerte Ventile (26, 28) umfasst, wobei die Ventileinheit (18) einen Fluss eines Fluids steuert; und
ein Schlauchset (2), welches einen proximalen Zweig und einen distalen Zweig aufweist, wobei der proximale Zweig durch die Ventileinheit (18) geöffnet und geschlossen wird, durch die Ventile (26, 28) hindurchtritt und mit zumindest einer Fluidquelle (60) verbunden ist, und wobei zumindest ein Teil (12) des distalen Zweigs flexibel ist und zum Einführen durch einen Operationsportkatheter konfiguriert ist, und wobei eine distale Spitze (14) des distalen Zweigs dazu konfiguriert ist, mit einem chirurgischen Werkzeug (16) innerhalb eines Operationsfeldes gegriffen und manipuliert zu werden.

2. System gemäß Anspruch 1, wobei die Spitze (14) eine starre Spitze ist.

3. System gemäß Anspruch 2, wobei die Spitze (14) eine Länge von nicht länger als 5,08 cm (2 Inches) aufweist.

4. System gemäß Anspruch 1, wobei die Spitze (14) eine poröse chirurgische Matte (70) aufweist.

5. System gemäß Anspruch 1, ferner umfassend eine Fernbedienung, durch welche der Benutzer das Steuermodul manipulieren kann.

6. System gemäß Anspruch 5, wobei die Fernbedienung aus der Gruppe von Fernbedienungen, bestehend aus Sprachsteuerungsbedienungen, Fußpedalen, pneumatisch aktivierten Steuerungen, Funksteuerungen und berührungsempfindlichen Handsteuerungen, ausgewählt ist.

7. System gemäß Anspruch 1, wobei das Ventil in der Ventileinheit (18) ein Magnetventil ist.

8. System gemäß Anspruch 7, wobei das Magnetventil ein Magnet-Quetschventil (52) umfasst.

9. System gemäß Anspruch 1, ferner umfassend eine Druckgasquelle (71), wobei der Fluss des Fluids druckbeaufschlagt ist.

10. System gemäß Anspruch 1, wobei die Spitze (14) des distalen Zweigs durch einen Roboterarm repositionierbar ist.

11. System gemäß Anspruch 1, wobei die Spitze (14) des distalen Zweigs durch ein chirurgisches Instrument (16) repositionierbar ist.

12. System gemäß Anspruch 1, ferner umfassend einen zweiten distalen Zweig, welcher an den proximalen Zweig gekoppelt ist.

13. System gemäß Anspruch 1, ferner umfassend ein Ventil, durch welches der distale Zweig verschlossen werden kann.

14. Fluidsteuerventil zur Absaugung, Insufflation und Spülung eines Operationsfeldes, wobei das Ventil dadurch charakterisiert ist, dass es Folgendes umfasst:
eine durch einen Benutzer gehandhabte Fernsteuereinheit;
eine flexible Fluidleitung (12), welche zum Einführen durch einen Operationsportkatheter konfiguriert ist, zwischen einer Fluidquelle und dem Operationsfeld verläuft, und durch das Ventil, welches den Durchfluss des Fluids durch ein Lumen (13) der Fluidleitung steuert, geöffnet und geschlossen wird;
eine Schnittstelle zwischen dem Ventil und der Fluidleitung, welches für den Benutzer während der Handhabung zugänglich ist; und
wobei das Ventil außerhalb eines sterilen Operationsfeldes angeordnet ist und durch die Fernsteuereinheit ferngesteuert ist.

15. Fluidsteuerventil gemäß Anspruch 14, wobei das Ventil ein Quetschventil (26) ist.

16. Fluidsteuerventil gemäß Anspruch 14, wobei die Fluidquelle eine aus den Fluidquellen, bestehend aus Vakuumquellen, Druckgasquellen, Kohlestoffdioxidquellen, Spülflüssigkeitsbehältern und Salzlösungsversorgungen ausgewählte Fluidquelle ist.

17. Fluidsteuerventil gemäß Anspruch 14, ferner umfassend ein zweites Quetschventil (28), welches einen Fluidfluss durch eine zweite Fluidleitung steuert.

## Revendications

1. Système servant à faire entrer et sortir des fluides dans / d'un champ chirurgical, le système étant **caractérisé en ce qu'**il comprend :
un module de commande manipulé par un utilisateur ;
une unité de vannes (18) commandée à l'extérieur dudit champ chirurgical et comprenant deux vannes (26, 28) commandées par ledit utilisateur via ledit module de commande, ladite unité de vannes (18) réglant le débit d'un fluide ; et
un ensemble de tubes (2) doté d'une branche proximale et d'une branche distale, ladite branche proximale étant ouverte et fermée par ladite unité de vannes (18), passant par lesdites vannes (26, 28) et étant reliée à au moins une source de fluide (60), et au moins une partie (12) de ladite branche distale étant souple et conçue pour s'insérer à travers un port chirurgical, et une pointe distale (14) de ladite branche distale étant conçue pour être saisie et manipulée par un outil chirurgical (16) è l'intérieur d'un champ chirurgical.

2. Système selon la revendication 1, dans lequel ladite pointe (14) est une pointe rigide.

3. Système selon la revendication 2, dans lequel ladite pointe (14) a une longueur ne dépassant pas 5.08 cm (deux pouces).

4. Système selon la revendication 1, dans lequel ladite pointe (14) comprend un tapis chirurgical poreux (70).

5. Système selon la revendication 1, comprenant également une télécommande par lequel ledit utilisateur manipule ledit module de commande.

6. Système selon la revendication 5, dans lequel ladite télécommande est choisie dans le groupe de télécommandes constitué de dispositifs de commande vocale, pédales, commandes pneumatiques, commandes transmis sans fils et dispositifs de commande manuels à effleurement.

7. Système selon la revendication 1, dans lequel ladite vanne dans ladite unité de vannes (18) est une électrovanne.

8. Système selon la revendication 7, dans lequel ladite électrovanne (14) comprend une électrovanne à pincement (52).

9. Système selon la revendication 1, comprenant également une alimentation en gaz comprimé (71) par lequel ledit débit de fluide est comprimé.

10. Système selon la revendication 1, dans lequel ladite pointe (14) de ladite branche distale est conçue pour être repositionnée par un bras robotisé.

11. Système selon la revendication 1, dans lequel ladite pointe (14) de ladite branche distale est conçue pour être repositionnée par un outil chirurgical (16).

12. Système selon la revendication 1, comprenant également une deuxième branche distale accouplée à ladite branche proximale.

13. Système selon la revendication 1, comprenant également une vanne par laquelle ladite branche distale peut être occluse.

14. Vanne de réglage de fluide pouvant être utilisée pour l'aspiration, l'insufflation et l'irrigation d'un champ chirurgical, ladite vanne étant **caractérisée en ce qu'**elle comprend :
une unité de télécommande actionnée par un utilisateur ;
une conduite souple de fluide (12) conçue pour s'insérer à travers un port chirurgical, s'étendant entre une source de fluide et ledit champ chirurgical, et étant ouverte et fermée par ladite vanne de réglage du passage de fluide par une lumière (13) de ladite conduite de fluide ;
une interface entre ladite vanne et ladite conduite de fluide étant accessible audit utilisateur pendant la marche ; et
ladite vanne étant située à l'extérieur d'un champ chirurgical stérile et étant télécommandée par ladite unité de télécommande.

15. Vanne de réglage de fluide selon la revendication 14, dans laquelle ladite vanne est une vanne à pincement (26).

16. Vanne de réglage de fluide selon la revendication 14, dans laquelle ladite source de fluide est une source de fluide choisie parmi les sources de fluide consistant en sources de vide, sources de gaz comprimé, sources d'anhydride carbonique, réservoirs de liquide et alimentations de solutions saines.

17. Vanne de réglage de fluide selon la revendication 14, comprenant également une deuxième vanne à pincement (28) pour le réglage du débit de fluide à travers une deuxième conduite de fluide.
